# EUROPEAN PATENT APPLICATION

(11) **EP 4 618 047 A1**
(43) Date of publication of application: **17.09.2025**
(21) Application number: 25719605.5
(22) Date of filing: 24.01.2025
(51) Int. Cl.: G06V 30/186, G06V 30/19, G06V 30/182, G06N 3/08, G06N 3/04

(54) **SYSTEM, METHOD, AND PROGRAM FOR RECOGNIZING POLYMER MOLECULAR STRUCTURAL FORMULA BY USING ARTIFICIAL INTELLIGENCE**

(30) Priority: 24.01.2024 KR 20240010891
(71) Applicant: LG Management Development Institute Co., Ltd., Seoul 07336 (KR)
(72) Inventor: LEE, Seungjun, Gimpo-si, Gyeonggi-do 10099 (KR); JO, Ahra, Gimpo-si, Gyeonggi-do 10113 (KR); LEE, Soonyoung, Seoul 06299 (KR)
(74) Representative: BCKIP Part mbB
(86) International application number: PCT/KR2025/001495
(87) International publication number: WO 2025/159578

(57) **Abstract**

A system, method, and program for recognizing a polymer molecular structural formula that use artificial intelligence are disclosed. The system for recognizing the polymer molecular structural formula using artificial intelligence includes at least one processor, and at least one memory storing a command or information that cause the at least one processor to perform an operation, wherein the operation performed by the command or the information includes detecting a polymer molecular structural formula image to generate detection data including information about a bracket and a subscript, and inputting the detection data to each of a first model and a second model to output first cluster data from the first model and to output second cluster data including group information about the bracket and the subscript and including information different from the first cluster data from the second model.

## Description

### [Technical Field]

The present invention relates to a system, a method, and a program for recognizing a polymer molecular structural, and more particularly, to a system, a method, and a program that can recognize a molecular structural formula from a polymer molecular structural formula image using artificial intelligence.

### [Background Art]

A structural formula refers to a graphical representation of a chemical structure or a molecular structure. The structural formula may show how atoms are arranged in a three-dimensional space. The structural formula may also indicate clearly or implicitly chemical bonding of molecules.

A polymer refers to a substance having a high molecular weight, which is formed by the linking of a plurality of monomers that are molecules having a low molecular weight. When the polymer is represented by a molecular structural formula, the polymer is denoted by attaching an arbitrary number of times (n, m, etc., a subscript) of polymerization to a repeated monomer.

Meanwhile, the molecular structural formula is provided in the form of an image in various documents, papers, etc. Since the molecular structural formula is provided in the form of the image, it is difficult to identify or select the molecular structural formula through general search. In particular, since the polymer may be a copolymer including two or more different types of monomers, it is required to recognize a polymer molecular structural formula image.

### [Disclosure]

### [Technical Problem]

An object of the present invention is directed to providing a system, a method, and a program that can recognize an accurate polymer molecular structure by selecting and recognizing necessary information from a polymer molecular structural formula image.

Objects of the present invention are not limited to the above-described object, and other objects that are not mentioned will be clearly understood by those skilled in the art from the following description.

### [Technical Solution]

A system for recognizing the polymer molecular structural formula using artificial intelligence includes at least one processor, and at least one memory storing a command or information that cause the at least one processor to perform an operation, wherein the operation performed by the command or the information includes detecting a polymer molecular structural formula image to generate detection data including information about a bracket and a subscript, and inputting the detection data to each of a first model and a second model to output first cluster data from the first model and to output second cluster data including group information about the bracket and the subscript and including information different from the first cluster data from the second model.

The polymer may include two or more different types of monomers.

The first cluster data may include at least one of class information and coordinate information of the bracket and the subscript.

The second model may include a metric function.

In various embodiments, the detecting the polymer molecular structural formula image to generate the detection data including the information about the bracket and the subscript may include inputting the polymer molecular structural formula image into a detector to output the detection data, and the detector may include a detection transformer (DETR) or a deformable DETR.

The detection data may include an embedding vector.

In addition, at least one of the first model and the second model may include a matrix including the embedding vector.

The group information about the bracket and the subscript may include information about which bracket and subscript among the detected brackets and subscripts are included in a group corresponding to which monomer.

In various embodiments, the operation performed by the command or the information may further include converting the structural formula image into a predetermined string format including simplified molecular input line entry system (SMILES) based on cluster data and outputting the converted structural formula image.

In addition, the operation performed by the command or the information may further include acquiring information about a plurality of atomic regions from the polymer molecular structural formula image and acquiring information about bonding relationships between a plurality of atoms based on the information about the plurality of atomic regions.

A method for recognizing the polymer molecular structural formula using artificial intelligence, performed by at least one processor according to another aspect of the present invention, include detecting a polymer molecular structural formula image to generate detection data including information about a bracket and a subscript, and inputting the detection data to each of a first model and a second model to output first cluster data from the first model and to output second cluster data including group information about the bracket and the subscript and including information different from the first cluster data from the second model.

The polymer may include two or more different types of monomers.

The group information about the bracket and the subscript may include information about which bracket and subscript among the detected brackets and subscripts are included in a group corresponding to which monomer.

The outputting the second cluster data from the second model may include generating a matrix that projects the detection data into another feature space.

A program stored in a computer-readable recording medium according to another aspect of the present invention may be stored in a computer-readable recording medium to execute the method for recognizing the polymer molecular structural formula according to embodiments of the present invention.

### [Advantageous Effects]

According to embodiments of the present invention, by simultaneously including a first model that outputs class information and coordinate information from detection data of brackets and subscripts of a polymer molecular structural formula and a second model that outputs group information about the brackets and the subscripts, it is possible to more accurately recognize a copolymer molecular structural formula including two or more different types of monomers.

Effects of the present invention are not limited to the above-described effects, and other effects that are not mentioned will be clearly understood by those skilled in the art from the following description.

### [Description of Drawings]

FIG. 1 is a schematic diagram of a system that can implement a method for recognizing a polymer molecular structural formula according to one embodiment of the present invention.
FIG. 2 is a block diagram for describing a configuration of a device that performs the method for recognizing the polymer molecular structural formula according to one embodiment of the present invention.
FIG. 3 is a flowchart for describing the method for recognizing the polymer molecular structural formula according to embodiments of the present invention.
FIG. 4 is a block diagram for describing the method for recognizing the polymer molecular structural formula according to embodiments of the present invention.
FIG. 5 shows molecular structural formulas for illustratively describing the method for recognizing the polymer molecular structural formula according to embodiments of the present invention.
FIG. 6 is a schematic diagram illustratively describing metric learning that may be used in the method for recognizing the polymer molecular structural formula according to embodiments of the present invention.
FIG. 7 is a diagram for describing a structural formula image according to embodiments of the present invention.
FIG. 8 is a diagram for describing an atomic region recognition model according to embodiments of the present invention.
FIG. 9 is a diagram for describing information about a plurality of atomic regions according to embodiments of the present invention.
FIG. 10 is a diagram for describing a method for acquiring information about bonding relationship according to embodiments of the present invention.
FIG. 11 is a diagram for describing a bonding relationship recognition model according to embodiments of the present invention.

### [Modes of the Invention]

The following embodiments are provided as examples so that the spirit of the present invention can be sufficiently conveyed to those skilled in the art to which the present invention pertains. Therefore, the present invention is not limited to the embodiments described below and may be embodied in other forms.

The same reference numerals refer to the same components throughout the present invention. The present invention does not describe all elements of the embodiments, and common content in the art to which the present invention pertains or content that overlaps between the embodiments will be is omitted. Terms "unit," "module," "member," and "block" used in the specification may be implemented as software or hardware, and according to the embodiments, a plurality of "units," "modules," "members," and "blocks" may be implemented as one component, or one "unit," "module," "member," and "block" may also include a plurality of components.

Throughout the present specification, when a certain portion is described as being "connected" to another portion, this includes not only a case in which the portions are directly connected, but also a case in which they are indirectly connected or in which another component exists therebetween, and the form of "connection" includes both wired and wireless connections and includes all cases in which the portions are directly or indirectly connected in any manner.

In addition, when a certain portion is described as "including" a certain component, it means further including another component rather than precluding another component unless specifically stated otherwise.

Throughout the present specification, when a first member is described as being positioned "on" a second member, this includes both a case in which the first member is in contact with the second member and a case in which a third member is present between the two members.

Terms such as first and second are used to distinguish one component from another, and the components are not limited by the above-described terms.

A singular expression includes plural expressions unless the context clearly dictates otherwise.

In each operation, identification symbols are used for convenience of description, and the identification symbols do not describe the sequence of each operation, and each operation may be performed in a different sequence from the specified sequence unless a specific sequence is clearly described in context.

A system for recognizing a molecular structural formula according to the present invention may include a device, and the device may include all kinds of devices that can perform computational processing and provide results to a user. For example, the system for recognizing the molecular structural formula according to the present invention may include at least one of a computer, a server device, and a portable terminal, or may be implemented in any one form having the same or similar functions thereof.

In addition, the system for recognizing the molecular structural formula according to the present invention may include being implemented in the form of a service provided to a user terminal in the form of a web service or the like from a server, or in the form in which the server and the user terminal are linked.

However, the method for implementing the system for predicting the properties of a material according to the present invention is not limited thereto, and all forms of systems that can be implemented may be included in the present invention.

*Here, the computer may include, for example, a notebook, a desktop, a laptop, a tablet PC, a slate PC, etc., which are equipped with a web browser.

The server device is a server that processes information in communication with an external device, and may include an application server, a computing server, a database server, a file server, a game server, a mail server, a proxy server, and a web server.

The portable terminal is, for example, a wireless communication device ensuring portability and mobility and may include all kinds of handheld-based wireless communication devices such as a personal communication system (PCS), a global system for mobile communications (GSM), a personal digital cellular (PDC), a personal handyphone system (PHS), a personal digital assistant (PDA), international mobile telecommunication-2000 (IMT-2000),
code division multiple access-2000 (CDMA-2000), w-code division multiple access (W-CDMA), a wireless broadband internet (WiBro) terminal, a smart phone, and wearable devices such as a watch, a ring, a bracelet, an anklet, a necklace, glasses, contact lenses, or a head-mounted device (HMD).

AI models according to embodiments of the present invention may be controlled, executed, learned, driven, etc. by at least one processor, and therefore, at least one of the tasks of executing, learning, and driving the AI models may be performed by at least one processor. The AI models may be stored in a memory.

In addition, according to embodiments of the present invention, a command that causes at least one processor to perform an operation may be included in at least one memory. The at least one processor may cause the AI model to operate, and may also cause other components (e.g., a conversion unit, a calculation unit, etc.) that implement the system to operate in addition to the AI model.

In addition, in the embodiments, the AI model may include an artificial neural network (ANN), a machine learning model, etc.

The ANN is a model used in machine learning, and may refer to a model having problem-solving capability, which is composed of artificial neurons (nodes) forming a network by synaptic connections. The ANN may be defined by connection patterns between neurons of different layers, a learning process that updates model parameters, and an activation function that generates an output value.

The ANN may include an input layer, an output layer, and optionally, one or more hidden layers. Each layer may include one or more neurons, and the ANN may include the synapses that connect the neurons to the neurons. In the ANN, each neuron may output a function value at an activation function for input signals input through the synapses, weights, and bias.

Parameters may include the model parameters and hyperparameters, and the model parameters refer to parameters that are changed and determined through learning and may include the weights of the synaptic connections, the biases of the neurons, etc. The hyperparameters refer to parameters that need to be set before learning in a machine learning algorithm and include a learning rate, the number of iterations, a mini-batch size, an initialization function, etc.

The learning of the ANN may include determining the model parameters that minimize a loss function, where the loss function may be used as an indicator for determining optimal model parameters in the learning process of the ANN.

The machine learning may include supervised learning, unsupervised learning, reinforcement learning, or the like according to a learning method but is not limited thereto.

Among the ANNs, the machine learning implemented with a deep neural network (DNN) including a plurality of hidden layers is called deep learning, and the deep learning is included as a part of the machine learning.

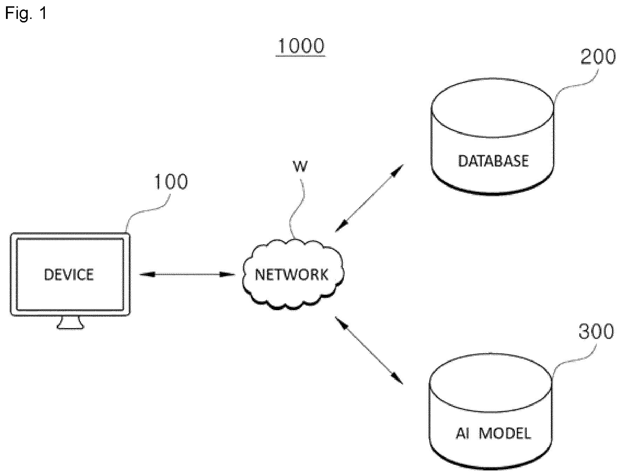

FIG. 1 is a schematic diagram of a system that can implement a method for recognizing a polymer molecular structural formula according to one embodiment of the present invention.

As shown in FIG. 1, a system 1000 may include a device 100, a database 200, and an AI model 300.

The device 100, the database 200, and the AI model 300 that are included in the system 1000 may perform communication via a network W. Here, the network W may include a wired network and a wireless network. For example, the network may include various networks such as a local area network (LAN), a metropolitan area network (MAN), and a wide area network (WAN).

In addition, the network W may also include the well-known world wide web (WWW). However, the network W according to the present invention is not limited to the above-listed networks and may include, at least in part, a well-known wireless data network, a well-known telephone network, or a well-known wired and wireless television network.

The device 100 may acquire or output feature data on information about a polymer molecular structural formula based on the AI model 300. The feature data may include a feature vector, embedding, or the like.

The database 200 may store various training data for training the AI model 300. In addition, the database 200 may store a polymer molecular structural formula image, a ground-truth (GT) value, a training set for training, etc., and in various embodiments, may store output data output by the AI model 300. However, the system 1000 may not include the database 200 when the training of the AI model 300 is completed.

FIG. 1 shows a case in which the database 200 is implemented outside the device 100. In this case, the database 200 may be connected to the device 100 in a wired or wireless communication manner. However, this is only one embodiment, and the database 200 may also be implemented as one component of the device 100.

FIG. 1 shows a case in which the AI model 300 is implemented outside the device 100 (e.g., implemented in a cloud-based manner), but is not limited thereto, and may be implemented as one component of the device 100.

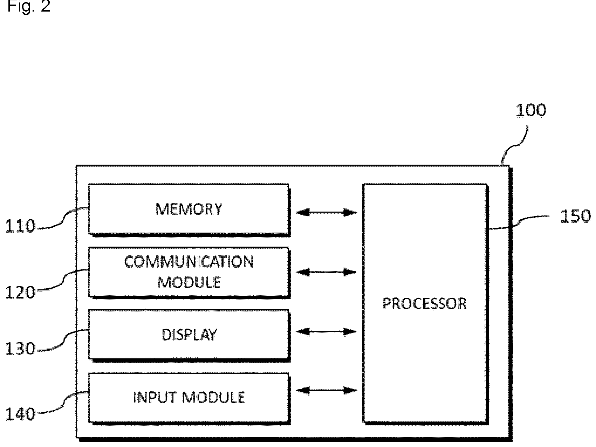

FIG. 2 is a block diagram for describing a configuration of a device that performs the method for recognizing the polymer molecular structural formula according to one embodiment of the present invention.

As shown in FIG. 2, the device 100 may include a memory 110, a communication module 120, a display 130, an input module 140, and a processor 150. However, the present invention is not limited thereto, and software and hardware components of the device 100 may be modified/added/omitted according to a required operation within a scope obvious to those skilled in the art. In addition, the device 100 may be replaced with a system, and the device 100 may include a plurality of devices and in this case, each component included in the device 100 may be included in at least one of the plurality of devices.

The memory 110 may store data supporting various functions of the device 100 and a program for the operation of the processor 150, store input/output data, and store a plurality of application programs or applications that are driven on the present device, data, command and the AI model for the operation of the device 100. At least some of the application programs may be downloaded from an external server via wireless communication. The memory 110 may store a command or information that causes the processor 150 to perform an operation.

Such memory 110 may include at least one type of storage medium among a flash memory type, a hard disk type, a solid state disk type (SSD type), a silicon disk drive type (SDD type), a multimedia card micro type, a card-type memory (e.g., an SD or XD memory), a random access memory (RAM), a static random access memory (SRAM), a read-only memory (ROM), an electrically erasable programmable ROM (EEPROM), a programmable ROM (PROM), a magnetic memory, a magnetic disk, and an optical disk.

In addition, the memory 110 may include a database that may be separate from the present device and connected in a wired or wireless communication manner. The database 200 shown in FIG. 1 may be implemented as one component of the memory 110.

The communication module 120 may include one or more components that enable communication with an external device, and may include at least one of, for example, a broadcasting reception module, a wired communication module, a wireless communication module, a short-range communication module, or a position information module.

The wired communication module may include not only various wired communication modules such as a LAN module, a WAN module, and a value added network (VAN) module, but also various cable communication modules such as a universal serial bus (USB), a high definition multimedia interface (HDMI), a digital visual interface (DVI), a recommended standard 232 (RS-232), power line communication, and a plain old telephone service (POTS).

In addition to the WiFi module and the wireless broadband (WiBro) module, the wireless communication module may include a wireless communication module for supporting various wireless communication methods such as global system for mobile communication (GSM), code division multiple access (CDMA), wideband CDMA (WCDMA), universal mobile telecommunications system (UMTS), time division multiple access (TDMA), long term evolution (LTE), 4G, 5G, or 6G.

The display 130 displays (outputs) information or data that are processed in the device 100, data that are input or output through the AI model 300, etc. In addition, the display 130 may display execution screen information of an application program (e.g., an application) driven on the device 100, or user interface (UI) or graphic user interface (GUI) information according to such execution screen information.

The input module 140 is for receiving information from a user, and when receiving information through a user input unit, the processor 150 may control the operation of the device 100 to correspond to the input information.

Such input module 140 may include a hardware physical key (e.g., a button located on at least one of a front surface, a back surface, and a side surface of the present device, a dome switch, a jog wheel, a jog switch, etc.) and a software touch key. As an example, the touch key may be formed as a virtual key, a soft key, or a visual key that is displayed on a touchscreen type of the display 130 through software processing or may be formed as the touch key disposed a portion other than the touchscreen. Meanwhile, the virtual key or visual key may have various forms and may be displayed on the touchscreen, and may be formed as, for example, a graphic, text, an icon, a video, or a combination thereof.

The processor 150 may be implemented with a memory that stores data for an algorithm for controlling the operations (including learning or execution of the AI model) of the components in the device 100 or a program that reproduces the algorithm, and at least one processor (not shown) that performs the above-described operation using the data stored in the memory. In this case, the memory and the processor may each be implemented as separate chips or may be implemented as a single chip.

The processor 150 may be one or more processors and/or processing circuits for executing program codes and controlling basic operations of the device 100. The processor may include a central processing unit (CPU), a graphic processing unit (GPU), a field-programmable gate array (FPGA), an application-specific integrated circuit (ASIC), a complex programmable logic device (CPLD), a dedicated circuit for implementing functions, a special-purpose processor for implementing neural networkbased processing, or a system including other systems.

In one embodiment, the system 1000 or the device 100 according to the present invention may include at least one processor, and when including a plurality of processors, the plurality of processors may be included in different devices 100.

In addition, the processor 150 may control the operations of the components in the device 100 by combining any one or a plurality of the above-described components in order to implement various embodiments according to the present invention, which will be described below, on the device 100.

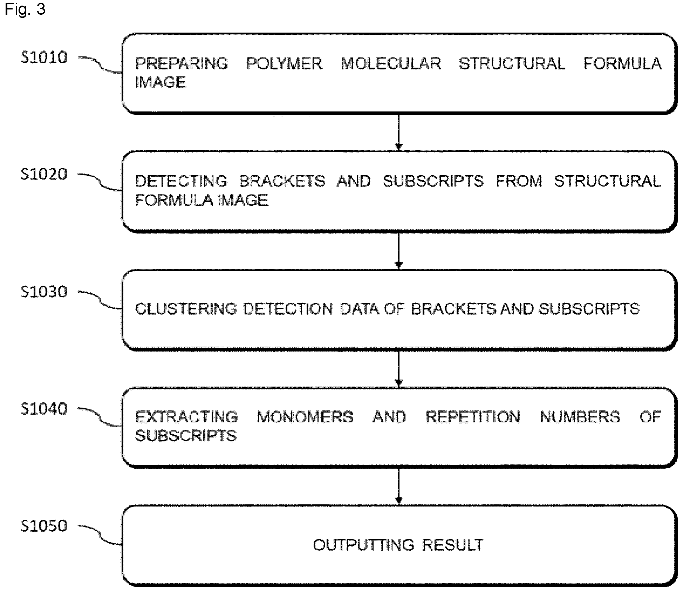

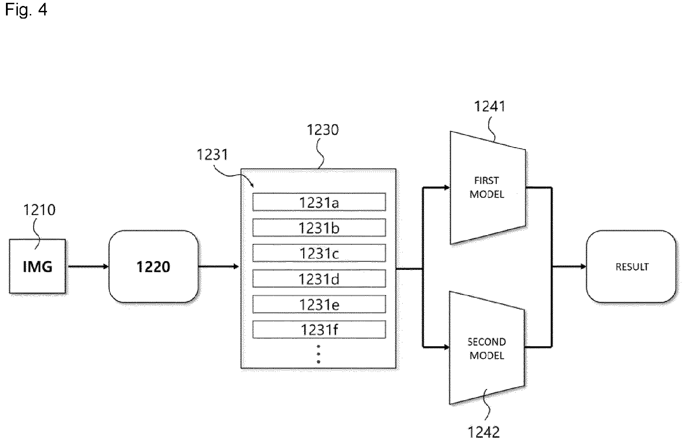

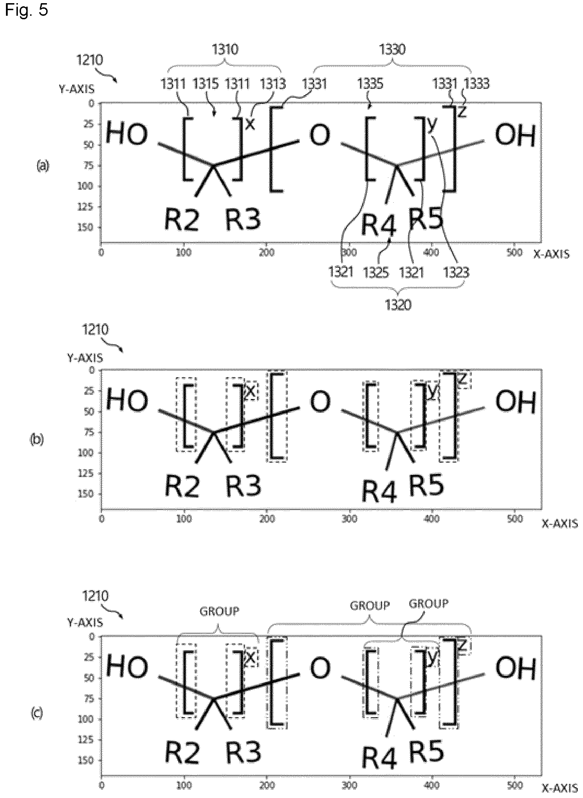

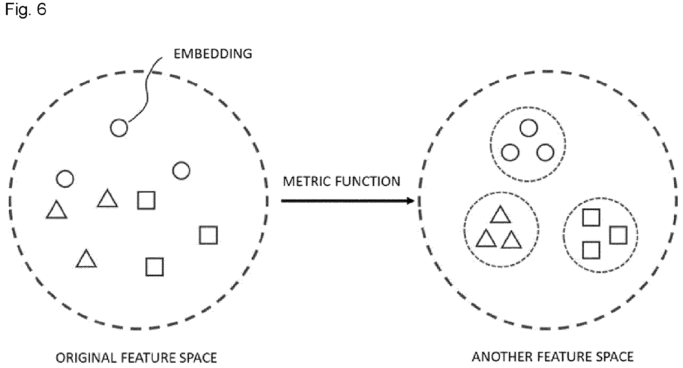

FIG. 3 is a flowchart for describing the method for recognizing the polymer molecular structural formula according to embodiments of the present invention, FIG. 4 is a block diagram for describing the method for recognizing the polymer molecular structural formula according to embodiments of the present invention, and FIG. 5 shows molecular structural formulas for illustratively describing the method for recognizing the polymer molecular structural formula according to embodiments of the present invention. FIG. 6 is a schematic diagram illustratively describing metric learning that may be used in the method for recognizing the polymer molecular structural formula according to embodiments of the present invention.

Referring to FIGS. 3 and 4, the method for recognizing the polymer molecular structural formula according to embodiments includes preparing a polymer molecular structural formula image (S1010), detecting brackets and subscripts from the polymer molecular structural formula image to generate detection data (S1020), clustering the brackets and the subscripts in the detection data to generate cluster data (S1030), extracting monomers and repetition numbers of subscripts using the data detected from the polymer molecular structural formula image (S1040), and outputting a result (S1050).

The method for recognizing the polymer molecular structural formula according to embodiments may be performed by at least one processor. In addition, commands or information that cause the at least one processor to perform an operation may be included in at least one memory. The at least one processor or the at least one memory may be included in a plurality of devices. In addition, the method for recognizing the polymer molecular structural formula according to embodiments may be performed by the operation performed by the commands or information and may include performing iterative learning.

Hereinafter, each operation will be described in detail.

First, the method for recognizing the polymer molecular structural formula according to embodiments may include preparing a polymer molecular structural formula image 1210 (S1010). The polymer molecular structural formula image 1210 may include the polymer molecular structural formula including at least one monomer and may include a copolymer molecular structural formula including two or more different types of monomers. For example, as shown in FIG. 5A, the polymer molecular structural formula image 1210 may include the copolymer molecular structural formula including three types of monomers 1315, 1325, and 1335.

The preparing the polymer molecular structural formula image 1210 may be performed by at least one processor, and the commands or the information cause the at least one processor to perform the operation may be stored in at least one memory. The preparing the polymer molecular structural formula image 1210 may include storing the polymer molecular structural formula image 1210 in a memory included in the processor, a separate volatile or non-volatile memory, or the like. The polymer molecular structural formula image 1210 may include an image stored in the memory, an image received from the outside through a network or a communication module and stored, an image received from the outside through the network or the communication module in real time and streamed, etc. However, the present invention is not limited thereto and may include all methods and approaches in which the polymer molecular structural formula image is prepared so that the polymer molecular structural formula image 1210 may be recognized through the operation performed by at least one processor.

The polymer molecular structural formula image 1210 may be an image in which the structural formula is represented in a graphical form. The structural formula may refer to a graphical representation of a chemical structure or a molecular structure. The structural formula may include information about the arrangement of atoms in a three-dimensional space and may include information about chemical bonding between the atoms. The polymer molecular structural formula image 1210 may include an annotation, and for example, the annotation may refer to a name of a compound, etc.

Referring to the example of FIG. 5, as shown in FIG. 5A, the polymer molecular structural formula image 1210 includes first to third monomers 1315, 1325, and 1335, and each monomer is represented by brackets 1311, 1321, and 1331 and subscripts 1313, 1323, and 1333 that refer to the number of repetition of the corresponding monomer. The polymer molecular structural formula image 1210 may include a first group 1310 including the first monomer 1315, the brackets 1311 distinguishing the first monomer 1315, and the subscript 1313, and may further include second and third groups 1320 and 1330 that include the second monomer 1325 and the third monomer 1335, respectively.

Here, the bracket refers to a notation for defining or distinguishing a unit of a repeated monomer in a polymer. That is, the molecule inside the brackets represents a structure of each monomer, and a line coming out of the brackets represents how the monomer is bonded to other atoms or molecules. The subscript refers to the number of repetition of the monomer, and when characters such as n or m are used as the subscript, it represents that the monomer is repeated several times.

Next, the brackets and the subscripts are detected from the polymer molecular structural formula image 1210 (S1020).

Referring to FIGS. 3 and 4, the detecting the brackets and the subscripts from the polymer molecular structural formula image 1210 may include detecting the polymer molecular structural formula image 1210 and outputting detection data 1230 about the brackets and the subscripts. The detection data 1230 may include information about the brackets and the subscripts. In addition, the detection data 1230 about the brackets and the subscripts may include at least one embedding 1231, and the embedding may include an embedding vector that is the form of a vector.

The detecting the polymer molecular structural formula image 1210 may include inputting the image into a detector 1220 and outputting the detection data 1230 about the brackets and the subscripts. The detector 1220 may include an object detector of transformer series and include a detection transformer (DETR) or a deformable DETR, but the present invention is not limited thereto.

Referring to the example of FIG. 5, as shown in FIG. 5B, the brackets (dotted lines) and the subscripts (dash-dotted lines) may each be detected by detecting the polymer molecular structural formula image 1210.

Meanwhile, each of the at least one embedding 1231 included in the detection data 1230 about the brackets and the subscripts may be an embedding including each individual information of the polymer molecular structural formula image 1210. The embedding 1231 may include the embedding vector including information about the bracket and the embedding vector including information about the subscript. The embedding vector may be, for example, the embedding vector of a length of 1024.

The embedding 1231 may include individual embedding including information about each of the brackets 1311, 1321, 1331 and the subscripts 1313, 1323, 1333 that refer to the number of repetition of the corresponding monomer, and the individual embedding may include the embedding vector. The embedding 1231 may each include one embedding vector including information about a left bracket and another embedding vector including information about a right bracket, which constitute the brackets.

Referring to the example of FIG. 5, the polymer molecule included in the polymer molecular structural formula image 1210 shown in FIG. 5A may include the first to third brackets 1311, 1321, and 1331 and the first to third subscripts 1313, 1323, and 1333, and the information about each of the brackets and the subscripts may each correspond to separate embedding vectors.

For example, in embodiments, the embedding 1231 may include an embedding vector 1231a including information about the left first bracket 1311, an embedding vector 1231b including information about the right first bracket 1311, and an embedding vector 1231c including information about the first subscript 1313. In addition, the embedding 1231 may include embedding vectors 1231d and 1231e that include information about each of the left and right brackets 1321 of the second group 1320 and an embedding vector 1231f including information about the second subscript 1323. In addition, the embedding 1231 may include embedding vectors 1231d and 1231e that include information about each of the left and right brackets 1321 of the second group 1320 and an embedding vector 1231f including information about the second subscript 1323.

Meanwhile, the detecting the brackets and the subscripts from the polymer molecular structural formula image 1210 may include recognizing remaining parts other than the brackets and the subscripts in the polymer molecular structural formula image, and may detect the atoms constituting the polymer molecule included in the polymer molecular structural formula image 1210 and bonding relationships between the atoms. In addition, the detecting the atoms constituting the polymer molecule included in the polymer molecular structural formula image 1210 and the bonding relationships between the atoms may include detecting the atoms constituting the monomer and the bonding relationships between the atoms.

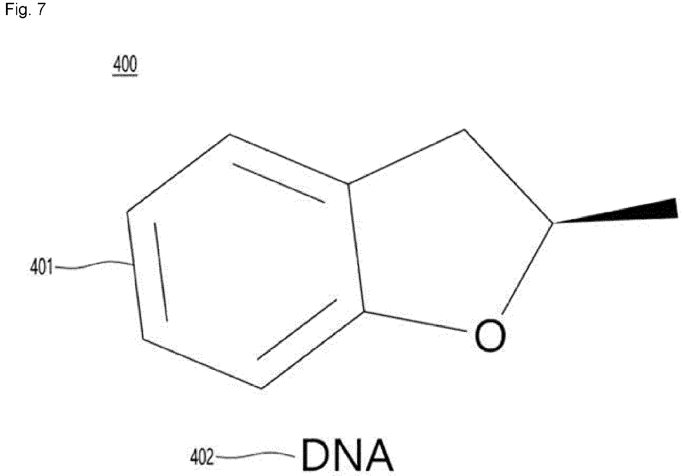

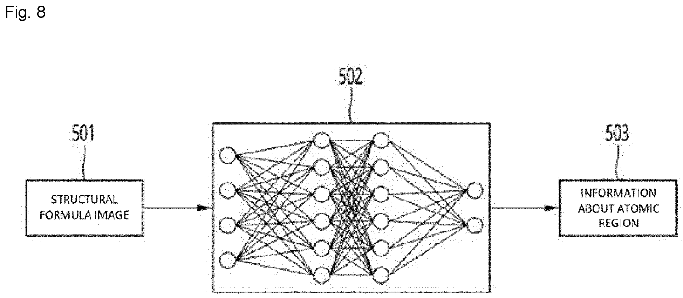

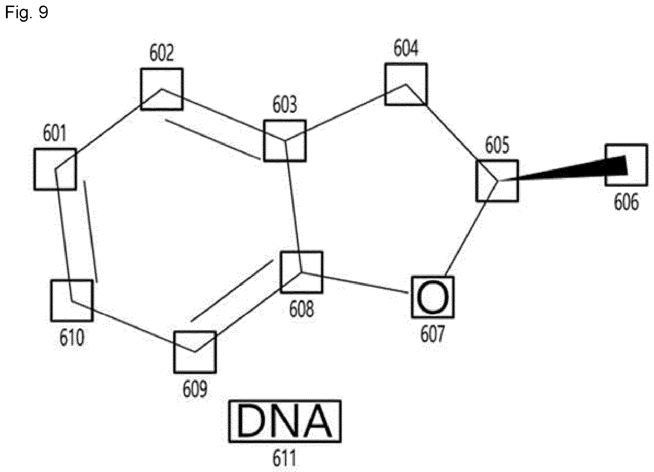

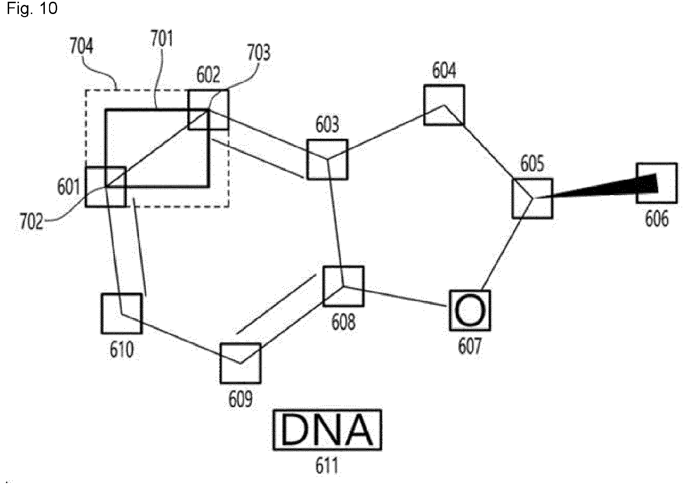

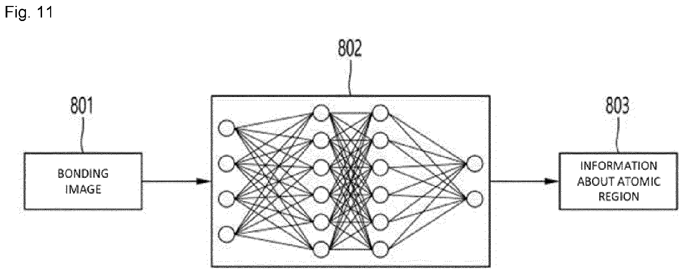

A method for detecting the atoms constituting the polymer molecule included in the polymer molecular structural formula 1210 and the bonding relationships between the atoms according to various embodiments of the present invention will be described in more detail with reference to FIGS. 7 to 11. FIG. 7 is a diagram for describing a structural formula image according to embodiments of the present invention, FIG. 8 is a diagram for describing an atomic region recognition model according to embodiments of the present invention, FIG. 9 is a diagram for describing information about a plurality of atomic regions according to embodiments of the present invention, FIG. 10 is a diagram for describing a method for acquiring information about bonding relationship according to embodiments of the present invention, and FIG. 11 is a diagram for describing a bonding relationship recognition model according to embodiments of the present invention.

First, referring to FIG. 7, a structural formula image 400 may include a structural formula 401 that graphically represents a molecular structure. In addition, the structural formula image 400 may include an annotation 402. When the structural formula image 400 is generated, there is a case in which an annotation may be imaged and included as a description or a name of the molecular structure in the structural formula image. Therefore, when recognizing the structural formula image 400 and converting the structural formula image 400 into a predetermined string format, it is necessary to filter the structural formula 401 alone as a recognition target, excluding the annotation 402 included in the structural formula image 400.

The information about the atomic region may include at least one of identification information about the atomic region, information about an atomic position, and information about the atom in the structural formula image. The identification information about the atomic region may refer to identification information (or a number) that may distinguish each of a plurality of atomic regions recognized in the structural formula image.

In addition, the information about the atomic position may refer to coordinate information corresponding to the atomic region in the structural formula image. For example, when the atomic region is represented as a quadrangle, the information about atomic position may include a coordinate of each vertex of the quadrangle corresponding to the atomic region and a coordinate of a center point of the quadrangle.

In addition, the information about the atom may include information about an element symbol of the atom corresponding to the atomic region. For example, when an image corresponding to the atomic region is represented as a vertex, carbon (C) may correspond to the information about the atom. In addition, when the element symbol (e.g., oxygen (O)) is described in the image corresponding to the atomic region, information about the corresponding element symbol (O) may correspond to the information about the atom.

Meanwhile, the information about the atomic region may include information about reliability of the information about atomic region acquired from the structural formula image. For example, the processor may acquire the information about reliability of information about the atomic region acquired from the structural formula image as a value between 0.00 and 1.00, and may use only the information about the atomic region having the information about reliability equal to or greater than a predetermined value.

Referring to FIG. 8, the processor may input a structural formula image 501 to an atomic region recognition model 502 and acquire the information about the atomic region output from the atomic region recognition model 502.

The atomic region recognition model 502 may be an artificial neural network (ANN) trained to output information about at least one atomic region 503 included in the image with respect to the input structural formula image 501. The ANN is a model used in machine learning, and may refer to a model having problem-solving capability, which is composed of artificial neurons (nodes) forming a network by synaptic connections. For example, the atomic region recognition model 502 may be an ANN model based on a convolutional neural network (CNN).

The processor may train the atomic region recognition model 502 composed of the ANN using learning data about the structural formula image and the information about the atomic region. Meanwhile, the atomic region recognition model 502 may be a model trained by the processor.

The trained atomic region recognition model 502 may be stored in a memory or stored in a storage unit of a server. The processor may cause the model stored in the memory, etc. to perform an operation.

Meanwhile, referring to FIG. 9, the processor may acquire information about a plurality of atomic regions 601, 602, 603, 604, 605, 606, 607, 608, 609, 610, and 611 that are output from the atomic region recognition model 502. The information about a plurality of atomic regions may include information about vertex regions 601, 602, 603, 604, 605, 606, 608, 609, and 610 in the structural formula, an element symbol region 607 in which the element symbol is described, and an annotation region 611. For example, since the atomic region recognition model 502 may be trained to output the element symbol region 607 in which the element symbol is described, there may also be a case in which the atomic region recognition model 502 outputs the annotation region 611 as the information about the atomic region. Therefore, there may be a need to filter the information about the annotation region from the information about the plurality of atomic regions. For example, since the annotation region 611 is not shown to have a bonding relationship with other atomic regions, when there is no bonding relationship between the annotation region 611 and other atomic regions, the processor 180 may classify the annotation region 611 as an annotation. Meanwhile, the processor may acquire information about bonding relationships between the plurality of atoms based on the information about the plurality of atomic regions.

The processor may acquire the information about the bonding relationship of each atomic region with another atomic region based on the information about the plurality of atomic regions. The processor may acquire a bonding image between a first atom and a second atom based on first atomic region information and second atomic region information among the information about the plurality of atomic regions, and acquire the information about the bonding relationship of the first atom and the second atom based on the acquired bonding image.

Referring to FIG. 10, a method for acquiring the information of the bonding relationship will be described, in which the processor may select the first atomic region information 301 among the information about the plurality of atomic regions. **In** addition, the processor may select the second atomic region information 602 different from the first atomic region information. **In** addition, the processor may acquire a bonding image 701 between the first atom and the second atom based on first atomic position information 702 of the first atomic region information 601 and second atomic position information 703 of the second atomic region information 602. In this case, the first atomic position information 702 and the second atomic position information 703 may be a center point position of each the atomic region. However, the first atomic position information 702 and the second atomic position information 703 are not limited to the center point positions of the atomic regions.

The processor may acquire the bonding image 701 including the center point position of each atomic region based on the first atomic position information 702 of the first atomic region information 601 and the second atomic position information 703 of the second atomic region information 602. In addition, the processor may acquire a bonding image 704 including the first atomic region and the second atomic region based on the first atomic region information 601 and the second atomic region information 602. A size and shape of the bonding image may be variously adjusted.

Meanwhile, the processor may acquire the bonding images between combinable atoms for each of the plurality of atomic regions. However, when acquiring the bonding images between all combinable atoms, the amount of computation may increase. Therefore, the processor may acquire only the bonding image between the first atom and the second atom that are positioned within a predetermined distance from each other based on the information about the plurality of atomic regions.

For example, the processor may select the second atomic region positioned within a predetermined distance from the first atomic region based on the first atomic region information among the information about the plurality of atomic regions. Referring to FIG. 10, the processor may specify the second atomic regions 602, 603, 608, 609, and 610 that are positioned within a predetermined distance from the first atomic region 601 and acquire the bonding image between the first atom 601 and each of the second atoms to acquire the information about the bonding relationships between the first atom and the second atoms.

In addition, the processor may determine that third atomic regions 604, 605, 606, 607, and 611 that are positioned outside a predetermined distance from the first atomic region 601 have no bonding relationship. Therefore, the amount of computation may be decreased.

Meanwhile, the processor may acquire the information about the bonding relationships between the atoms based on the acquired bonding images. For example, the processor may input the bonding images into a bonding relationship recognition model and acquire the information about the bonding relationships output from the bonding relationship recognition model.

FIG. 11 is a diagram for describing the bonding relationship recognition model, and referring to FIG. 11, the processor may input a bonding image 801 into a bonding relationship recognition model 802 and acquire the information about the bonding relationship output from the bonding relationship recognition model 802.

The information about the bonding relationship is information about bonding between the atoms and may include information about non-bonding, single bonding, double bonding, triple bonding, up-direction bonding, down-direction bonding, etc. The non-bonding may refer to a case in which there is no bonding between the atoms. The up-direction bonding may refer to a bonding coming forward in a plane, which is indicated by a wedge line. In addition, the down-direction bonding may refer to a bonding going backward in a plane, which is indicated by a dash line. The bonding relationship recognition model 802 may be an artificial neural network (ANN) trained to output information about the bonding relationships 803 with respect to the input bonding image 801. The ANN is a model used in machine learning, and may refer to a model having problem-solving capability, which is composed of artificial neurons (nodes) forming a network by synaptic connections. For example, the bonding relationship recognition model 802 may be the ANN model based on a convolutional neural network (CNN).

The processor may train the bonding relationship recognition model 802 composed of the ANN using learning data about the bonding images and the information about the bonding relationships.

The trained bonding relationship recognition model 802 may be stored in a memory. The processor may use the bonding relationship recognition model 802 stored in the memory.

Meanwhile, the processor may generate an adjacency matrix based on the information about the plurality of atomic regions and the information about the bonding relationships between the plurality of atoms. The processor may generate the adjacency matrix with each of the plurality of atoms as a vertex and with the information about the bonding relationship of each of the plurality of atoms as an edge.

FIG. 11 is a diagram for describing the adjacency matrix, and referring to FIG. 11, the processor may generate each of the atoms of the plurality of atomic regions 601 to 611 as the vertex of the adjacency matrix. Meanwhile, the processor may generate the adjacency matrix with the information about the bonding relationship of each of the plurality of atoms as the edge. For example, the processor may generate an edge value at the adjacency matrix by mapping each of the information about the bonding relationship to an arbitrary number. For example, the processor may map the non-bonding to '0', the single bonding to '1', the double bonding to '2', and the triple bonding to '3'. In addition, the processor may map a case in which the bonding is in an upward direction in the order of a row and a column with the row as a starting vertex and the column as an arrival vertex in order to represent the directionality of the up-direction bonding, to '5', and map a case in the opposite direction to '0'.

For example, referring to FIG. 11, since a value at the sixth column of the fifth row of the adjacency matrix is the up-direction bonding from a first atom 605 to a second atom 606, a corresponding value of '5' may be generated. Meanwhile, a value at the fifth column of the sixth row of the adjacency matrix, which is the opposite direction, may be generated as '0'. Similarly, the processor 180 may map a case in which the bonding is in a downward direction in the order of the row and the column with the row as a starting vertex and the column as an arrival vertex in order to represent the directionality of the down-direction bonding, to '6', and map a case in the opposite direction to '0'.

Meanwhile, the processor may generate a predetermined string format corresponding to the structural formula image based on the generated adjacency matrix. The processor may acquire the information about the bonding relationships with other atoms by traversing each of the atomic regions corresponding to the vertices of the adjacency matrix. The processor may specify the information about the atom of each of the plurality of atomic regions using the acquired information about the bonding relationships between the atoms and generate a predetermined string format corresponding to the structural formula image based on the information about the plurality of atoms and the information about the bonding relationships between the plurality of atoms.

In this case, the string format may include a mol file format, an sdf file format, etc. that are file formats that may represent information about compounds (e.g., element positions, bonding relationships, etc.). Meanwhile, the string format may include information about a simplified molecular input line entry system (SMILES).

Meanwhile, the method for detecting the atoms of the polymer molecule, the bonding relationships between the atoms, etc. from the polymer molecular structural formula image according to embodiments of the present invention is not limited to the descriptions with reference to FIGS. 7 to 11, and various methods for detecting the structural formula image may all be used.

Furthermore, in the method for recognizing the polymer molecular structural formula according to embodiments of the present invention, the detecting the atoms constituting the polymer molecule and the bonding relationships between the atoms that are included in the polymer molecular structural formula image 1210 is not necessarily included in the operation of detecting the brackets and the subscripts from the polymer molecular structural formula image 1210, and the detecting the atoms constituting the polymer molecule and the bonding relationships between the atoms may be included in another operation or implemented as a separate operation.

Referring back to FIGS. 3 and 4, subsequently, cluster data is generated by clustering the brackets and the subscripts in the detection data (S1030).

The generating the cluster data by clustering the brackets and the subscripts in the detection data may include each inputting the detection data into a first model 1241 and a second model 1242 to output first cluster data including at least one of class information and coordinate information of the brackets and the subscripts from the first model 1241 and to output second cluster data including group information about the brackets and the subscripts from the second model 1242. The cluster data may include the first cluster data and the second cluster data.

The first model 1241 and the second model 1242 may be pre-set algorithms or pre-trained models. At least one of the first model 1241 and the second model 1242 may be executed or trained by the processor.

The first cluster data output from the first model 1241 may include information different from the second cluster data output from the second model 1242.

In embodiments, the detection data including the embedding 1231 may be input into the first model 1241 so that the class information and the coordinate information of an object (a bracket, a subscript) corresponding to each embedding 1231 may be output. For example, referring to FIG. 5A, the polymer molecular structural formula image 1210 may be projected on a space having an x-axis and a y-axis, and each detection object (a bracket, a subscript, an atom, an atomic bonding, etc.) of the polymer molecule may have a coordinate according to the x-axis and y-axis of the space.

The first model 1241 may include a linear layer composed of a matrix. In one embodiment, the first model 1241 may include the linear layer including the matrix (e.g., 1024 × 4) that derives class probabilities for four classes of the bracket, the subscript, the atom, and the carbon (C) from the embedding 1231 and the linear layer including the matrix (e.g., 1024 × 4) that derives the coordinate information of the detection object such as the bracket and the subscript from the embedding 1231.

The class information output from the first model 1241 may include classification information of the object corresponding to each embedding 1231, that is, information about what type of object the object refers to. For example, when the embedding 1231 about the brackets 1311, 1321, and 1331 is input into the first model 1241 and related information is output, the class information indicating that information about the corresponding embedding 1231 corresponds to 'bracket' may be output.

By outputting the first cluster data including the class information and the coordinate information of the bracket and the subscript through the first model 1241, information about which monomer the bracket and the subscript correspond to, where the bracket and the subscript are positioned in the bonding relationships between other atoms, etc. may be output.

In embodiments, the detection data including the embedding 1231 may be input to the second model 1242 so that group information of objects (a bracket, a subscript) corresponding to each embedding 1231 may be output. The group information may include information about which the brackets and the subscript among the brackets and the subscripts form a monomer group.

The second model 1242 may include a layer that rearranges the input embedding 1231 into another new space. The second model 1242 may include the matrix (e.g., 1024 × 1024) that projects the input embedding 1231 into the embedding of a different length (e.g., 1024 length). The second model 1242 may include a metric function.

Referring to FIG. 6, the second model 1242 including the metric function may enable the embeddings of an original feature space to be clustered (grouped) in a new feature space. Therefore, the second model 1242 may include generating the matrix that projects the detection data into a new different feature space.

The second model 1242 may be a model trained by various methods and may be a model trained based on, for example, an infoNCE loss, but is not limited thereto.

For example, the second model 1242 may be executed or trained in a manner that assigns a loss by defining a positive pair and a negative pair using the infoNCE loss. The positive pair corresponds to ground truth, and for example, the first brackets 1311 and the first subscript 1313 included in the first group 1310 corresponding to the first monomer 1315 may output the result as the positive pair. On the other hand, when clustering into one group such as the first brackets 1311 and the second subscript 1323 is not the ground truth, the corresponding negative result is output.

By outputting the second cluster data including the group information about the bracket and the subscript through the second model 1242, information about which brackets and subscripts among the detected brackets and subscripts are included in a group corresponding to which monomer may be output. For example, referring to FIG. 5C, the second model 1242 may output the second cluster data including information that the first brackets 1311 and the first subscript 1313 are one group included in the first group 1310 corresponding to the first monomer 1315, and may output the second cluster data including similar group information for the brackets and the subscripts included in the remaining second and third groups 1320 and 1330.

As described above, according to embodiments of the present invention, the system using the method for recognizing the polymer molecular structural formula according to the present invention may accurately recognize the copolymer molecular structural formula including two or more different types of monomers by simultaneously including the first model 1241 that outputs the class information and the coordinate information from the detection data of the brackets and the subscripts of the polymer molecular structural formula and the second model 1242 that outputs the group information about the brackets and the subscripts.

Referring back to FIGS. 3 and 4, the monomers and repetition numbers of the subscripts that are detected from the polymer molecular structural formula image are extracted (S1040), and a result is output (S1050).

The extracting the monomers and the repetition numbers of the subscripts that are detected from the polymer molecular structural formula image may extract the monomers and the repetition numbers of the subscripts from information detected through the method of detecting the atoms constituting the polymer molecule and the bonding relationships between the atoms, but the present invention is not limited thereto and the monomers and the repetition numbers of the subscripts may be detected through various methods for recognizing the molecular structural formula.

In embodiments, the extracting the monomers and the repetition numbers of the subscripts that are detected from the polymer molecular structural formula image may use the method described with reference to FIGS. 7 to 11, but the present invention is not limited thereto.

The outputting the result (S1050) may generate a predetermined string format corresponding to the polymer molecular structural formula image based on the information about the plurality of atoms, the information about the bonding relationships between the plurality of atoms, and the cluster data.

In this case, the string format may include a mol file format, an sdf file format, etc. that are file formats that may represent information about compounds (e.g., element positions, bonding relationships, monomer information, etc.). Meanwhile, the string format may include information about the simplified molecular input line entry system (SMILES).

Meanwhile, the method for recognizing the polymer molecular structural formula according to embodiments of the present invention may be implemented by the system described with reference to FIGS. 1 and 2.

In addition, the disclosed embodiments may be implemented in the form of a recording medium in which computer-executable commands are stored. The commands may be stored in the form of program code, and when executed by the processor, program modules are generated to perform operations of the disclosed embodiments. The recording medium may be implemented as a computer-readable recording medium.

The computer-readable recording medium includes all types of recording media in which computer-decodable commands are stored. For example, there may be the ROM, the RAM, a magnetic tape, a magnetic disk, a flash memory, an optical data storage device, and the like.

As described above, the disclosed embodiments have been described with reference to the accompanying drawings. Those skilled in the art to which the present invention pertains will understand that the present disclosure may be implemented in different forms from the disclosed embodiments without departing from the technical spirit or essential features of the present invention. The disclosed embodiments are illustrative and should not be construed as being limited.

## Claims

1. A system for recognizing a polymer molecular structural formula using artificial intelligence, the system comprising:
at least one processor; and
at least one memory storing a command or information that cause the at least one processor to perform an operation,
wherein the operation performed by the command or the information includes:
detecting a polymer molecular structural formula image to generate detection data including information about a bracket and a subscript; and
inputting the detection data to each of a first model and a second model to output first cluster data from the first model and to output second cluster data including group information about the bracket and the subscript and including information different from the first cluster data from the second model.

2. The system of claim 1, wherein the polymer includes two or more different types of monomers.

3. The system of claim 1, wherein the first cluster data includes at least one of class information and coordinate information of the bracket and the subscript.

4. The system of claim 1, wherein the second model includes a metric function.

5. The system of claim 1, wherein the detecting the polymer molecular structural formula image to generate the detection data including the information about the bracket and the subscript includes inputting the polymer molecular structural formula image into a detector to output the detection data, and
the detector includes a detection transformer (DETR) or a deformable DETR.

6. The system of claim 1, wherein the detection data includes an embedding vector.

7. The system of claim 6, wherein at least one of the first model and the second model includes a matrix including the embedding vector.

8. The system of claim 2, wherein the group information about the bracket and the subscript includes information about which bracket and subscript among the detected brackets and subscripts are included in a group corresponding to which monomer.

9. The system of claim 1, wherein the operation performed by the command or the information further includes converting the structural formula image into a predetermined string format including simplified molecular input line entry system (SMILES) based on cluster data and outputting the converted structural formula image.

10. The system of claim 1, wherein the operation performed by the command or the information further includes acquiring information about a plurality of atomic regions from the polymer molecular structural formula image and acquiring bonding relationship information between a plurality of atoms based on the information about the plurality of atomic regions.

11. A method for recognizing a polymer molecular structural formula using artificial intelligence, performed by at least one processor, the method comprising:
detecting a polymer molecular structural formula image to generate detection data including information about a bracket and a subscript; and
inputting the detection data to each of a first model and a second model to output first cluster data from the first model and to output second cluster data including group information about the bracket and the subscript and including information different from the first cluster data from the second model.

12. The method of claim 11, wherein the polymer includes two or more different types of monomers.

13. The method of claim 12, wherein the group information about the bracket and the subscript includes information about which bracket and subscript among the detected brackets and subscripts are included in a group corresponding to which monomer.

14. The method of claim 12, wherein the outputting the second cluster data from the second model includes generating a matrix that projects the detection data into another feature space.

15. A program stored in a computer-readable recording medium to execute the method of any one of claims 11 to 14 by being coupled to a computer.
